# EUROPEAN PATENT APPLICATION

(11) **EP 4 803 565 A1**
(43) Date of publication of application: **09.09.2026**
(21) Application number: 24885661.9
(22) Date of filing: 28.10.2024
(51) Int. Cl.: C08J 9/28, A61L 27/18, A61L 27/50, A61L 27/52, A61L 27/56, C08G 65/08, C08G 65/333, C08G 65/334, C08J 3/24, C08J 3/075

(54) **PRODUCTION METHOD FOR HYDROGEL HAVING POROUS STRUCTURE**

(30) Priority: 02.11.2023 JP 2023188947
(71) Applicant: The University of Tokyo, Bunkyo-ku, Tokyo 113-8654 (JP); Gellycle Co., Ltd., Tokyo 113-0033 (JP)
(72) Inventor: ISHIKAWA, Shohei, Tokyo 113-8654 (JP); SAKAI, Takamasa, Tokyo 113-8654 (JP); KATASHIMA, Takuya, Tokyo 113-8654 (JP); KAMATA, Hiroyuki, Tokyo 113-0033 (JP)
(74) Representative: Cabinet Becker et Associés
(86) International application number: PCT/JP2024/038345
(87) International publication number: WO 2025/094888

(57) **Abstract**

An object of the present invention is to provide a novel production method capable of producing a hydrogel material having a µm-scale porous structure through which substances such as cells can permeate by a simpler method.

It has been found that a biocompatible hydrogel having a µm-scale porous structure can be produced by employing a freeze-thaw process as a trigger for porous structuring for an initial hydrogel having a non-porous structure once generated while setting the total concentration of a raw material polymer in a solution within a predetermined range.

## Description

### Technical Field

The present invention relates to a production method for a biocompatible hydrogel having a three-dimensionally communicating porous structure.

### Background Art

In recent years, a hydrogel having a polymer network structure has characteristics such as excellent water holding ability and biocompatibility, and thus is a material expected to be applied not only to medical purposes such as artificial tissues, regenerative medical scaffolds, sealants, adhesion preventing materials, drug delivery substrates, and contact lenses, but also to various applications such as sensors and surface coating materials (for example, Non Patent Literature 1). In particular, it is promising in material development for tissue engineering applications to produce a gel having a three-dimensionally communicating porous structure having a µm-scale size.

However, conventionally, in order to obtain the µm-scale porous structure, it has been necessary to use a top-down method such as microfabrication of a hydrogel or a polymer structure produced in advance by lithography or the like, or to produce a polymer material using a polymer raw material insoluble in a solvent. Meanwhile, in the case of using a hydrophilic polymer raw material such as a polyethylene glycol (PEG) having biocompatibility, a simple production technique of a two-solution mixing system is used, but in this method, only a hydrogel having a small nm-scale porous structure can be produced, and the permeation of cells and substances having a µm-scale size is difficult.

### Citation List

### Non Patent Literature

Non Patent Literature 1: Sakai et al., Macromolecules, 41, 5379-5384, 2008

### Summary of Invention

### Technical Problem

Therefore, an object of the present invention is to provide a novel production method capable of producing a hydrogel material having a µm-scale porous structure through which substances such as cells can permeate by a simpler method.

### Solution to Problem

In order to solve such problems, the present inventors have found that it is possible to produce a biocompatible hydrogel having a µm-scale porous structure by improving a conventional hydrogel production method and adopting a freeze-thaw process as a trigger for porous structuring for an initial hydrogel having a non-porous structure once generated while setting the total concentration of a raw material polymer in a solution within a predetermined range, thereby completing the present invention. The production method of the present invention can produce a hydrogel having a µm-scale porous structure by a simple method without requiring special equipment or the like. Furthermore, the present inventors have also found that the resulting hydrogel not only has a µm-scale sponge-like three-dimensional network structure (porous structure), but also has characteristics in physical properties such as excellent substance permeability, low swelling, and improvement in strength as compared with a non-porous hydrogel formed from the same polymer unit.

That is, the present invention provides, in a representative aspect,
<1> a production method for a hydrogel having a porous structure, including the steps of
   a) preparing a first solution in which a compound A is dissolved in a first solvent;
   b) preparing a second solution in which a compound B is dissolved in a second solvent;
   c) obtaining an initial hydrogel having a non-porous structure by mixing the first solution and the second solution, the step including mixing the first solution and the second solution at a ratio such that a total polymer concentration (Cₜₒₜₐₗ) in a third solution obtained by mixing the first solution and the second solution is equal to or less than a higher one of two concentrations: a viscosity-increasing feature concentration (C_{A}) unique to the compound A or a viscosity-increasing feature concentration (C_{B}) unique to the compound B;
   d) obtaining a frozen hydrogel by subjecting the initial hydrogel to a freezing treatment; and
   e) obtaining a hydrogel having a porous structure by treating the frozen hydrogel at a temperature equal to or higher than a higher one of melting points of the first and second solvents,
      wherein each of the initial hydrogel and the hydrogel having the porous structure forms a gel obtained by crosslinking the compound A and the compound B with each other,
      each of the first and second solvents is an aqueous solvent; and
      the compounds A and B are bi-, tri-, tetra-, or octa-branched polyethylene glycols (PEG) having one or more nucleophilic functional groups or electrophilic functional groups in total in a side chain or at a terminal.

The present invention provides, in a preferred aspect,
<2> the production method according to the above <1>, wherein the compound A and/or the compound B has a weight average molecular weight of 4×10⁴ or less;
<3> the production method according to the above <1>, wherein the freezing treatment is performed at a temperature of -10°C or lower for 1 hour or more;
<4> the production method according to the above <1>, wherein when a transmittance of the initial hydrogel is X, a transmittance of the frozen hydrogel is Y, and a transmittance of the hydrogel having the porous structure is Z, a relational expression of $X > Z > Y$ is satisfied;
<5> the production method according to the above <1>, wherein when an equilibrium swelling degree of the hydrogel having the porous structure prepared such that Cₜₒₜₐₗ is 20 g/L is Q_{low} and an equilibrium swelling degree of the hydrogel having the porous structure prepared with any Cₜₒₜₐₗ is Q, a relational expression of $Q < 1.2 * {Q}_{low}$ is satisfied;
<6> the production method according to the above <1>, wherein when a breaking stress at elongation (σₘₐₓ) of the initial hydrogel is Sᵢₙᵢₜᵢₐₗ and a breaking stress at elongation of the hydrogel having the porous structure is S, a relational expression of $S > {S}_{initial}$ is satisfied;
<7> the production method according to the above <1>, wherein when an elongation rate at break (λₘₐₓ) of the initial hydrogel is Tᵢₙᵢₜᵢₐₗ and an elongation rate at break of the hydrogel having the porous structure is T, a relational expression of $T > {T}_{initial}$ is satisfied;
<8> the production method according to the above <1>, wherein when a Young's modulus (Eₘₐₓ) of the initial hydrogel is Uᵢₙᵢₜᵢₐₗ and a Young's modulus of the hydrogel having the porous structure is U, a relational expression of $U > {U}_{initial}$ is satisfied;
<9> the production method according to the above <1>, wherein fracture energy of the hydrogel having the porous structure is higher than fracture energy of the initial hydrogel;
<10> the production method according to the above <1>, wherein the compound A is a bi-, tri-, tetra-, or octa-branched PEG having two or more nucleophilic functional groups in total in a side chain or at a terminal; and
   the compound B is a bi-, tri-, tetra-, or octa-branched PEG having two or more electrophilic functional groups in total in a side chain or at a terminal; and
<11> the production method according to the above <1>, wherein the nucleophilic functional group is selected from the group consisting of a thiol group and an amino group, and the electrophilic functional group is selected from the group consisting of a maleimidyl group, an N-hydroxy-succinimidyl (NHS) group, and a sulfosuccinimidyl group.

### Advantageous Effects of Invention

According to the production method of the present invention, it is possible to simply and efficiently obtain a biocompatible hydrogel having a µm-scale porous structure by employing a freeze-thaw process as a trigger for porous structuring while setting the total concentration of a raw material polymer within a predetermined range. In particular, the production method of the present invention is advantageous in that it does not require use of special equipment or the like, and does not require the removal of an organic solvent or a porogen unlike a conventional gelation method.

Since the hydrogel obtained by the production method of the present invention forms a sponge-like, µm-scale three-dimensional network structure (porous structure), the hydrogel can be a material suitable for cell infiltration and adhesion. In addition, according to the production method of the present invention, it is also possible to impart bioabsorbability to the gel, and in this case, the gel does not remain in the body after tissue regeneration or repair, and this characteristic can enhance the sense of security of both the doctor and the patient. The characteristic is expected to be evaluated as very valuable advantages in the field of regenerative medicine.

### Brief Description of Drawings

Fig. 1 is a schematic view illustrating the scheme of a production method of the present invention and the internal structural change of a hydrogel in each step.
Fig. 2 is the confocal laser microscope image of a hydrogel produced at each total polymer concentration.
Fig. 3 illustrates the confocal laser microscope images of hydrogels produced at different freezing times.
Fig. 4 is the three-dimensional stack image of the confocal laser microscope image of a hydrogel produced at each total polymer concentration.
Fig. 5 is the photograph of a hydrogel produced at each total polymer concentration after immersion in India ink.
Fig. 6 is the confocal laser microscope image of a porous hydrogel immersed in a liquid containing 10 µm particles.
Fig. 7 is a graph illustrating the equilibrium swelling degree of a hydrogel produced at each total polymer concentration.
Fig. 8 illustrates the confocal laser microscope images before and after the swelling of a porous hydrogel.
Fig. 9 illustrates graphs illustrating the breaking stress, breaking elongation, and fracture energy of a hydrogel produced at each total polymer concentration.
Fig. 10 is a graph illustrating the evaluation results of the breaking stress and stress relaxation property of a hydrogel produced at each total polymer concentration.
Fig. 11 illustrates the confocal laser microscope images of various porous hydrogels on which cells are seeded. 20 µm and 100 µm indicate distances (depths) from the surface of a porous hydrogel on which cells are seeded, respectively.

### Description of Embodiments

Hereinafter, embodiments of the present invention will be described. The scope of the present invention is not limited by the following description, and the present invention may be modified, as appropriate, and implemented using configurations other than those given below as examples within the spirit of the invention.

### (1) Production Method for Porous Hydrogel

A production method of the present invention is a production method for a hydrogel having a porous structure, and includes the following steps a) to e):
a) preparing a first solution in which a compound A is dissolved in a first solvent;
b) preparing a second solution in which a compound B is dissolved in a second solvent;
c) obtaining an initial hydrogel having a non-porous structure by mixing the first solution and the second solution;
d) obtaining a frozen hydrogel by subjecting the initial hydrogel to a freezing treatment; and
e) obtaining a hydrogel having a porous structure by treating the frozen hydrogel at a temperature equal to or higher than a higher one of melting points of the first and second solvents.

Here, the step c) includes mixing the first solution and the second solution at a ratio such that a total polymer concentration (Cₜₒₜₐₗ) in a third solution obtained by mixing the first solution and the second solution is equal to or less than a higher one of two concentrations: a viscosity-increasing feature concentration (C_{A}) unique to the compound A or a viscosity-increasing feature concentration (C_{B}) unique to the compound B.

The hydrogel obtained by the production method of the present invention has a sponge-like three-dimensional network structure (porous structure) formed by the phase separation of a polymer as a constituent component in a production process (hereinafter, such a structure may be referred to as a "sponge-like porous structure"). The network size is characterized by being on the order of µm, typically 10 to 400 µm, much larger than the order of nm obtained in conventional hydrogels. In this hydrogel, a polymer unit is not observed in voids forming a porous structure. That is, the hydrogel as a whole does not have two or more regions where the polymer density is greatly different. Since the polymer unit is not present in the voids, when the hydrogel is compressed by an external force, water can be discharged like a so-called normal sponge. In the production process in the present invention, some or all of the polymer units to be used are no longer solvophilic. As a result, a phase separation structure containing a non-solvophilic polymer as a constituent component is obtained.

In the present specification, the "gel" is generally a dispersion system of a polymer having high viscosity and lost fluidity, and refers to a state having a relationship of G' ≥G" between a storage elastic modulus G' and a loss elastic modulus G". In particular, when a solvent contained in the gel is an aqueous solvent, the gel is referred to as a "hydrogel".

Hereinafter, the compounds A and B constituting the hydrogel of the present invention and various conditions in various steps will be described.

### 1-1. Compounds A and B (Gel Constituent Components)

The compounds A and B which are components for forming the hydrogel of the present invention are hydrophilic polymer species, and can form a network structure, particularly a three-dimensional network structure, by crosslinking of the polymer species with each other in the final hydrogel.

More specifically, the compounds A and B are polyethylene glycols (PEG) having a plurality of branches, such as a bi-, tri-, tetra-, or octa-branched polyethylene glycol, with a tetra-branched polyethylene glycol being particularly preferred. Such a gel including a tetra-branched polyethylene glycol backbone is generally known as a Tetra-PEG gel, and a network structure is constructed by an AB-type cross-end coupling reaction between two types of tetra-branched polymers each having an electrophilic functional group such as an active ester structure and a nucleophilic functional group such as an amino group at the terminal (Matsunaga et al., Macromolecules, Vol. 42, No. 4, pp. 1344-1351, 2009). The Tetra-PEG gel can be easily and in-situ produced by the simple two-solution mixing of each polymer solution. By using the Tetra-PEG technique, the gelation time can also be controlled by adjusting the pH and ionic strength at the time of hydrogel preparation. Since the obtained hydrogel contains the PEG as a main component, the hydrogel is also excellent in biocompatibility. The compounds A and B can be selected from the branched polyethylene glycol group according to the application and shape and the like of the final hydrogel.

In the production method of the present invention, the compounds A and B are crosslinked with each other to form a three-dimensional network structure and constitute a hydrogel. Therefore, the compounds A and B have two or more nucleophilic functional groups or electrophilic functional groups in total for being linked to each other by crosslinking in the side chain of the polyethylene glycol (PEG) skeleton or at the terminal thereof. In this case, the compound A may be a first polymer unit having two or more nucleophilic functional groups in total in a side chain or at a terminal, and the compound B may be a second polymer unit having two or more electrophilic functional groups in total in a side chain or at a terminal. Here, the total of the nucleophilic functional group and the electrophilic functional group is preferably 5 or more. These functional groups are preferably present at the terminal. The content of the first polymer unit (compound A) may be larger than the content of the second polymer unit (compound B), or the content of the second polymer unit may be larger than the content of the first polymer unit.

Examples of the nucleophilic functional group present in the compounds A and B include a thiol group (-SH) (also referred to as a sulfhydryl group), and an amino group, and those skilled in the art can appropriately use a known nucleophilic functional group. Preferably, the nucleophilic functional group is a thiol group or an amino group. The nucleophilic functional groups may be the same as or different from each other, but are preferably the same. When the functional groups are the same, reactivity with an electrophilic functional group that forms a crosslinking bond becomes uniform, and a hydrogel having a uniform three-dimensional structure is easily obtained.

As the electrophilic functional group present in the compounds A and B, a maleimidyl group or an active ester group can be used. Examples of the active ester group include an N-hydroxy-succinimidyl (NHS) group and a sulfosuccinimidyl group. Those skilled in the art can appropriately use other known electrophilic functional groups. Preferably, the electrophilic functional group is a maleimidyl group. The electrophilic functional groups may be the same as or different from each other, but are preferably the same. When the functional groups are the same, reactivity with a nucleophilic functional group that forms a crosslinking bond becomes uniform, and a hydrogel having a uniform three-dimensional structure is easily obtained.

In addition to the combination of the nucleophilic functional group and the electrophilic functional group, a covalent bond forming reaction known to those skilled in the art, such as a cycloaddition reaction with azide and alkyne, and other reactions called click chemistry, can also be appropriately used.

The molecular weight of the PEG used as the compounds A and B can be typically a weight average molecular weight of 4×10⁴ or less. The molecular weight can be preferably 5×10³ to 4×10⁴, more preferably 1×10⁴ to 4×10⁴, and still more preferably 1×10⁴ to 2×10⁴. In a preferred aspect, the compound A and/or the compound B has a weight average molecular weight of 4×10⁴ or less.

Preferred non-limiting specific examples of the PEG having a nucleophilic functional group at a terminal include a compound represented by the following expression (I) having four branches of a polyethylene glycol backbone and having a thiol group at a terminal.

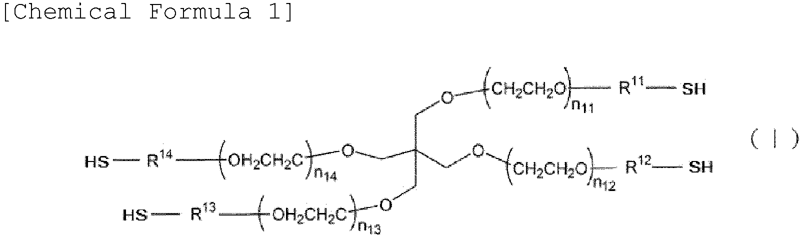

n₁₁ to n₁₄ may be the same as or different from each other. As the values of n₁₁ to n₁₄ are closer to each other, a uniform three-dimensional structure can be obtained, and the strength becomes higher. Therefore, in order to obtain a high-strength hydrogel, n₁₁ to n₁₄ are preferably the same. When the values of n₁₁ to n₁₄ are too high, the strength of the gel becomes weaker, and when the values of n₁₁ to n₁₄ are too low, the gel is hardly formed due to steric hindrance of the compound. Therefore, n₁₁ to n₁₄ are integer values of 28 to 227, preferably 56 to 227, and more preferably 56 to 114.

In the above expression (I), R¹¹ to R¹⁴ are linker moieties connecting a functional group and a core moiety. R¹¹ to R¹⁴ may be the same or different from each other, but are preferably the same in order to produce a high-strength hydrogel having a uniform three-dimensional structure. R1¹ to R¹⁴ represent a C₁ to C₇ alkylene group, a C₂ to C₇ alkenylene group, -NH-R¹⁵-, -CO-R¹⁵-, -R¹⁶-O-R¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, R¹⁶-NH-COR¹⁷-, or -R¹⁶-CO-NH-R¹⁷-. Here, R¹⁵ represents a C₁ to C₇ alkylene group. R1⁶ represents a C₁ to C₃ alkylene group. R¹⁷ represents a C₁ to C₅ alkylene group.

Here, the "C₁ to C₇ alkylene group" means an optionally branched alkylene group having 1 or more and 7 or less carbon atoms, and means a linear C₁ to C₇ alkylene group or a C₂ to C₇ alkylene group having one or two or more branches (the number of carbon atoms including the branch is 2 or more and 7 or less). Examples of the C₁ to C₇ alkylene group include a methylene group, an ethylene group, a propylene group, and a butylene group. Examples of C₁-C₇ alkylene groups include -CH₂-, -(CH₂)₂-, -(CH₂)₃-, - CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and - (CH₂)₃C(CH₃)₂CH₂-.

The "C₂ to C₇ alkenylene group" is a linear or branched alkenylene group having 2 to 7 carbon atoms and having one or two or more double bonds in the chain, and examples thereof include a divalent group having a double bond formed by excluding 2 to 5 hydrogen atoms of adjacent carbon atoms from the alkylene group.

Meanwhile, preferred non-limiting specific examples of the PEG having an electrophilic functional group at a terminal include a compound represented by the following expression (II) having four branches of a polyethylene glycol backbone and having a maleimidyl group at a terminal.

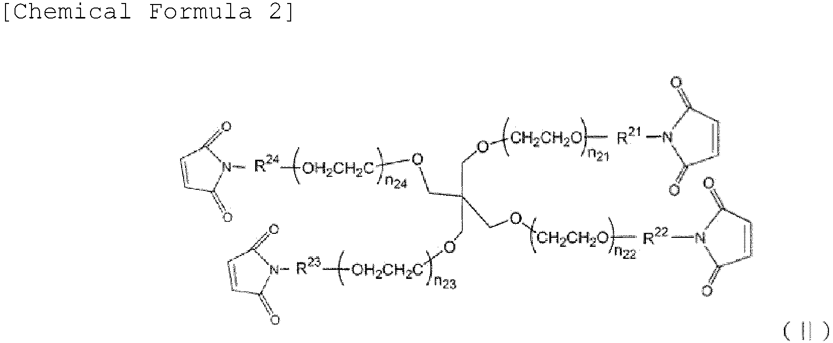

In the expression (II), n₂₁ to n₂₄ may be the same as or different from each other. As the values of n₂₁ to n₂₄ are closer to each other, the hydrogel can have a uniform three-dimensional structure and has high strength, which is preferable, and n₂₁ to n₂₄ are preferably the same. When the values of n₂₁ to n₂₄ are too high, the strength of the gel is weak, and when the values of n₂₁ to n₂₄ are too low, the gel is hardly formed due to steric hindrance of the compound. Therefore, n₂₁ to n₂₄ are integer values of 11 to 569, preferably 28 to 227, and more preferably 56 to 114.

In the expression (II), R²¹ to R²⁴ are linker moieties connecting a functional group and a core moiety. R²¹ to R²⁴ may be the same as or different from each other, but are preferably the same in order to produce a high-strength gel having a uniform three-dimensional structure. In the expression (II), R²¹ to R²⁴ may be the same as or different from each other, and represent a C₁ to C₇ alkylene group, a C₂ to C₇ alkenylene group, -NH-R²⁵-, -COR²⁵-, -R²⁶-O-R²⁷-, -R²⁶-NH-R²⁷-, -R²⁶-CO₂-R²⁷-, -R²⁶-CO₂-NH-R²⁷-, -R²⁶-CO-R²⁷-, -R²⁶-NH-CO-R²⁷-, or -R²⁶-CO-NH-R²⁷-. Here, R²⁵ represents a C₁ to C₇ alkylene group. R2⁶ represents a C₁ to C₃ alkylene group. R2⁷ represents a C₁ to C₅ alkylene group.

In the present specification, the alkylene group and the alkenylene group may have one or more arbitrary substituents. Examples of the substituent include an alkoxy group, a halogen atom (may be any of a fluorine atom, a chlorine atom, a bromine atom, and an iodine atom), an amino group, a mono- or disubstituted amino group, a substituted silyl group, an acyl group, and an aryl group, but are not limited thereto. When the alkyl group has two or more substituents, these substituents may be the same or different from each other. The same applies to alkyl moieties of other substituents including alkyl moieties (for example, alkyloxy groups and aralkyl groups and the like).

In the present specification, when a certain functional group is defined as "optionally substituted", the type of substituent, substitution position, and the number of substituents are not particularly limited, and when there are two or more substituents, these substituents may be the same or different from each other. Examples of the substituent include an alkyl group, an alkoxy group, a hydroxyl group, a carboxyl group, a halogen atom, a sulfo group, an amino group, an alkoxycarbonyl group, and an oxo group, but are not limited thereto. Further substituents may be present on these substituents.

### 1-2. Steps a) and b)

Steps a) and b) are steps of preparing first and second solutions containing compounds A and B, respectively. A solvent in the first and second solutions is typically water, but in some cases, may be a mixed solvent containing an alcohol such as a trace amount of ethanol, a polar solvent such as DMSO, and other organic solvents. The pH of the first and second solutions is in the range of 1.0 to 10.6, and more preferably in the range of 2.2 to 8.0.

As the pH in the first and second solutions, a pH buffer known in the art can be used. For example, the pH can be adjusted to the above range by using a citrate-phosphate buffer (CPB) and changing the mixing ratio of citric acid and disodium hydrogen phosphate. In the present specification, CPB and McIlvaine's buffer refer to the same.

The concentrations of the compounds A and B in the first and second solutions are adjusted such that the mixed solution (third solution) obtained in step c) described later has a predetermined concentration. The concentrations may be the same or different from each other as long as the concentration defined in step c) is satisfied, but the concentrations are preferably the same.

### 1-3. Step c)

Step c) is a step of preparing a third solution by mixing the first solution and the second solution, whereby the compounds A and B are crosslinked to obtain an initial hydrogel having a non-porous structure.

Here, the first solution and the second solution are mixed at a ratio such that a total polymer concentration (Cₜₒₜₐₗ) in the mixed solution (third solution) obtained by mixing the first solution and the second solution is equal to or less than a higher one of two concentrations: a viscosity-increasing feature concentration (C_{A}) unique to the compound A or a viscosity-increasing feature concentration (C_{B}) unique to the compound B.

In the present specification, the "viscosity-increasing feature concentration" refers to a concentration specific to each compound determined by the following procedure.

First, compound solutions having different concentrations (c) with CPB of 50 mM (pH 5) as a solvent are prepared. Next, the specific viscosity (ηsp) of each compound solution is determined. Here, ηsp can be obtained by a method common to those skilled in the art. The obtained ηsp is plotted as a Y axis, and c is plotted as an X axis. Linear fitting is performed on a region illustrating linearity on a low concentration side. When a specific viscosity at any concentration (c_arb) obtained from the fitted straight line is denoted by ηsp_fit, minimum c at which ηsp at c_arb satisfies the relationship of $ηsp > 1.2 \times ηsp_fit$ is defined as a viscosity-increasing feature concentration.

In a typical aspect, the total polymer concentration (Cₜₒₜₐₗ) in the third solution can be 90 g/L or less, preferably 40 g/L or less, more preferably 20 g/L, and more preferably 10 g/L.

The initial hydrogel formed at the stage of this step c) is gelled but does not have a µm-order porous structure.

As a means for mixing the first solution and the second solution, for example, a two-solution mixing syringe as disclosed in WO2007/083522 A can be used. The temperatures of the two solutions at the time of mixing are not particularly limited, and may be any temperature at which precursor units are each dissolved and the respective solutions have fluidity. For example, the temperatures of the solutions at the time of mixing may be in the range of 1°C to 100°C. The temperatures of the two solutions may be different from each other, but it is preferable that the temperatures are the same because the two solutions are easily mixed.

### 1-4. Step d)

Step d) is a step of obtaining a frozen hydrogel by subjecting the initial hydrogel having the non-porous structure obtained in step c) to a freezing treatment.

The freezing treatment in step d) can be performed using a method known in the art, and examples thereof include a cooling device capable of controlling a temperature to be equal to or lower than a freezing point and immersion in liquid nitrogen.

In the freezing treatment, the initial hydrogel is cooled to a temperature of preferably -10°C or lower, and more preferably -20°C or lower. A cooling time is not particularly limited as long as it is a period during which the initial hydrogel reaches a sufficiently frozen state, and can be appropriately set according to the capacity and size and the like of the initial hydrogel, but for example, cooling can be performed in a period of 1 hour or more, preferably 2 hours or more, and more preferably 3 hours or more. Typically, the freezing treatment in step d) is performed at a temperature of -20°C or lower for 24 hours or more.

### 1-5. Step e)

Step e) is a step of obtaining a hydrogel having a porous structure by treating the frozen hydrogel obtained in step d) at a temperature equal to or higher than a higher one of melting points of the first and second solvents.

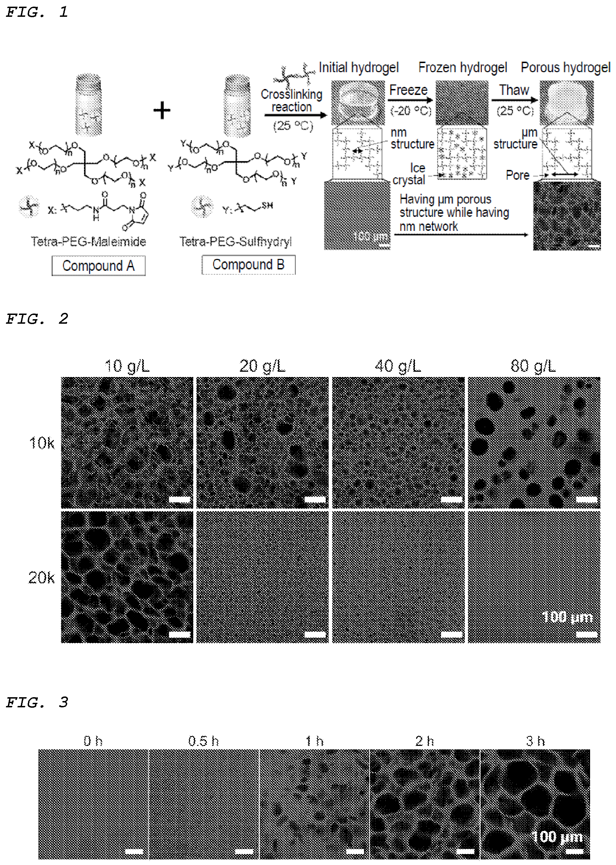

The production method of the present invention is characterized in that it has been found that a porous PEG gel having µm-order pores can be produced by simply subjecting the initial hydrogel having the non-porous structure to the freezing treatment in step d) and a subsequent thaw treatment in step e). Although not necessarily bound by theory, when the initial hydrogel is frozen below the freezing point as illustrated in Fig. 1, a solvent component (moisture) present in the gel is formed as ice crystals (in the figure, frozen hydrogel). The formation of the ice crystals increases the volume, resulting in the enrichment of the surrounding PEG network, which is the backbone of the gel. Next, when the gel is thawed at a temperature higher than the freezing point, a µm-order space (pore) is formed inside the gel, so that the hydrogel having the porous structure is obtained.

Here, "treatment at a temperature equal to or higher than the melting point of the solvent" in step e) means that any treatment is performed in which the frozen hydrogel exits from a frozen state and is brought into a thawed state where the solvent component in the gel can flow. Therefore, such a treatment includes placing the frozen hydrogel itself under an environment of the melting point or higher, or heating the frozen hydrogel with any device. The treatment also includes immersing the frozen hydrogel in a solvent having the melting point or higher (typically, water or a mixed solvent used as a solvent of the first and second solutions).

### (1) Characteristics of Porous Hydrogel, etc.

As described above, the hydrogel (porous hydrogel) having the porous structure obtained by the production method of the present invention has a µm-scale three-dimensional network structure (porous structure), and is a material suitable for cell infiltration and adhesion. Furthermore, the hydrogel has characteristics in physical properties such as excellent substance permeability, low swelling, and improvement in strength as compared with a non-porous hydrogel (initial hydrogel) formed from the same polymer unit.

The porous hydrogel obtained by the production method of the present invention has a transmittance lower than that of the non-porous initial hydrogel. This is because, in the porous hydrogel of the present invention, a polymer component as a constituent component behaves like the phase separation of a poorly soluble polymer in a solvent, and exhibits cloudiness rather than complete transparency. In terms of such a transmittance, it can be said that the hydrogel has characteristics completely different from those of a normal non-porous hydrogel that is substantially transparent.

More specifically, when the transmittance of the initial hydrogel obtained in step c) is X, the transmittance of the frozen hydrogel obtained in step d) is Y, and the transmittance of the porous hydrogel obtained in step e) is Z, the porous hydrogel obtained by the production method of the present invention satisfies
a relational expression of $X > Z > Y .$

The porous hydrogel obtained by the production method of the present invention is also characterized in terms of an equilibrium swelling degree (Q). Specifically, the porous hydrogel of the present invention is also characterized in terms of the equilibrium swelling degree. Specifically, when the equilibrium swelling degree of the hydrogel having the porous structure prepared such that the total polymer concentration (Cₜₒₜₐₗ) is 20 g/L is Q_{low} and the equilibrium swelling degree of the hydrogel having the porous structure prepared with an arbitrary total polymer concentration (Cₜₒₜₐₗ) is Q, a relational expression of $Q < 1.2 * {Q}_{low}$ is satisfied.

The porous hydrogel obtained by the production method of the present invention is also characterized in terms of a breaking stress at elongation (σₘₐₓ). Specifically, when the breaking stress at elongation (σₘₐₓ) of the initial hydrogel is Sᵢₙᵢₜᵢₐₗ and the breaking stress at elongation of the hydrogel having the porous structure is S, the porous hydrogel of the present invention satisfies
a relational expression of $S > {Si}_{nitial} .$

The porous hydrogel obtained by the production method of the present invention is also characterized in terms of an elongation rate at break (λₘₐₓ). Specifically, when the elongation rate at break (λₘₐₓ) of the initial hydrogel is Tᵢₙᵢₜᵢₐₗ, and the elongation rate at break of the porous hydrogel is T, the porous hydrogel of the present invention satisfies
a relational expression of $T > {T}_{initial} .$

The porous hydrogel obtained by the production method of the present invention is also characterized in terms of a Young's modulus (Eₘₐₓ). Specifically, when the Young's modulus (Eₘₐₓ) of the initial hydrogel is Uᵢₙᵢₜᵢₐₗ and the Young's modulus of the porous hydrogel is U, the porous hydrogel of the present invention satisfies
a relational expression of $U > {U}_{initial} .$

Furthermore, the fracture energy of the porous hydrogel obtained by the production method of the present invention is higher than the fracture energy of the initial hydrogel.

Those skilled in the art can understand that the transmittance, the equilibrium swelling degree, the breaking stress at elongation, the elongation rate at break, the Young's modulus, and the fracture energy described above can be measured using methods commonly used in the art.

The porous hydrogel obtained by the production method of the present invention can be processed into various shapes such as a thin film according to the applications. Any methods known in the art can be used for such processing. For example, in the case of the thin film, the thin film can be obtained by, for example, a method for applying a gelling solution onto a flat substrate such as glass in a state of having fluidity before complete solidification.

### Examples

Hereinafter, the present invention will be described in more detail with reference to Examples, but the present invention is not limited by these Examples. In the following Examples, the unit of g/L of a polymer concentration is used, and 1 g/L corresponds to 0.1% by weight.

### 1. Preparation of Non-Porous Hydrogel (Initial Hydrogel) Using Tetra-Branched Polyethylene Glycol (PEG)

As raw material polymers, Tetra-PEG-SH (tetrasulfhydryl-polyethylene glycol) having a -SH group at a terminal and Tetra-PEG-MA (maleimidyl-polyethylene glycol) having a maleimidyl group at a terminal were used. Commercially available products from NOF CORPORATION were used. The two types were used, each having a weight average molecular weight (Mw) of 10,000 or each having a weight average molecular weight of 20,000.

50 mM citrate-phosphate buffer (CPB) solutions having pH 5 in which Tetra-PEG-SH and Tetra-PEG-MA were respectively dissolved were prepared separately as a first solution and a second solution. Note that solutions having a plurality of PEG concentrations were prepared such that a total polymer concentration after mixing was in the range of 10 to 80 g/L.

The two solutions thus obtained were mixed in another container, and defoamed and stirred by a rotation-revolution mixer. Thereafter, the mixed solution was quickly transferred to a Falcon tube. The tube was capped to prevent drying and allowed to stand at room temperature for 24 hours.

A rheometer was used to observe time changes in a storage elastic modulus G' and a loss elastic modulus G" in the mixed solution (25°C, 1 Hz). It was confirmed that the gelled initial hydrogel was formed.

### 2. Freeze-Thaw Treatment

The initial hydrogel obtained above was subjected to a freezing treatment at -20°C for 0.5 to 24 hours to obtain a frozen product of the hydrogel. Unless otherwise specified, a freezing treatment time was 24 hours in all experiments. Thereafter, the frozen product was allowed to stand at 25°C for 24 hours to be thawed.

### 3. Evaluation of Internal Structure of Hydrogel

The internal structure of the obtained porous hydrogel was evaluated using a confocal laser microscope. Fig. 2 illustrates confocal laser microscope images of the porous hydrogels observed after the freeze-thaw process from initial solutions having total polymer concentrations (Cₜₒₜₐₗ) of 10, 20, 40, and 80 g/L after mixing the first and second solutions, using raw material PEGs with weight-average molecular weights of 10,000 (10 k) and 20,000 (20 k). As a result, it was found that the pore size of the resulting porous hydrogel becomes larger as the total polymer concentration (Cₜₒₜₐₗ) of the initial solution is lower. It was found that a porous structure is easily obtained even at higher Cₜₒₜₐₗ as the molecular weight of the raw material PEG is lower.

Similarly, the results of observing the internal structure of the hydrogel at a freezing time of 0 to 3 hours using the confocal laser microscope are illustrated in Fig. 3. As a result, it was found that when the freezing time exceeds 0.5 hours, a µm-order porous structure is obtained, and the tendency becomes more remarkable as the freezing time increases.

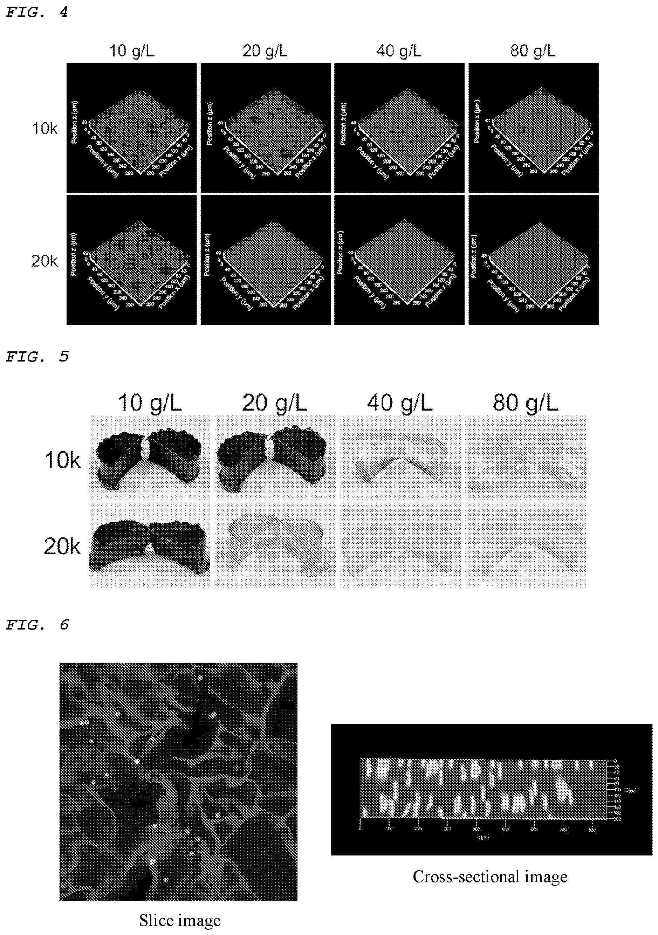

When the internal structure of the gel was three-dimensionally analyzed using the image of the confocal laser microscope, it was confirmed that the porous structure was a three-dimensionally communicating structure (Fig. 4).

### 4. Substance Permeability of Hydrogel

The obtained porous hydrogel was subjected to an immersion test in India ink to evaluate the substance permeability. A colloid size in the India ink had a distribution of 0.1 to 10 µm. The imaging images of the gel after immersion are illustrated in Fig. 5. As a result, it was found that the hydrogel having a larger pore size is colored black, and the India ink component permeates into the gel.

Similarly, an immersion test was performed using a suspension obtained by dispersing particles (Fluoresbrite^{®} YG Carboxylate Microspheres) having a size of 10 µm, which is equivalent to that of living cells, in D-PBS (-) at a concentration of 0.05% by weight, and the gel was observed with the confocal laser microscope. As a result, it was found that the particles are distributed inside the gel, and excellent permeability is exhibited (Fig. 6).

### 5. Evaluation of Swelling of Hydrogel

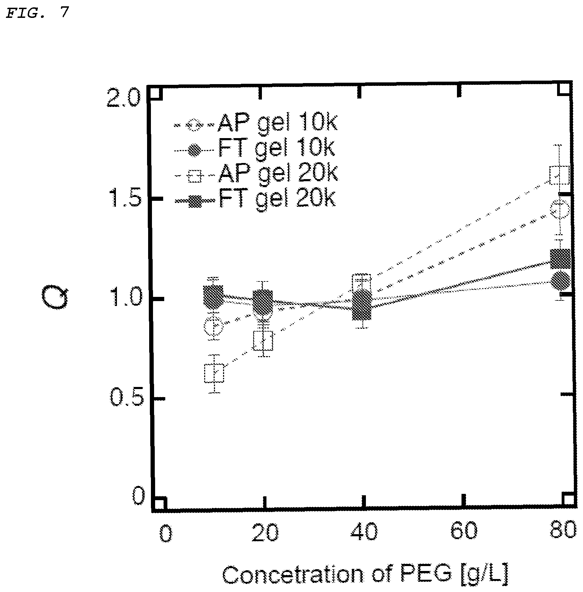

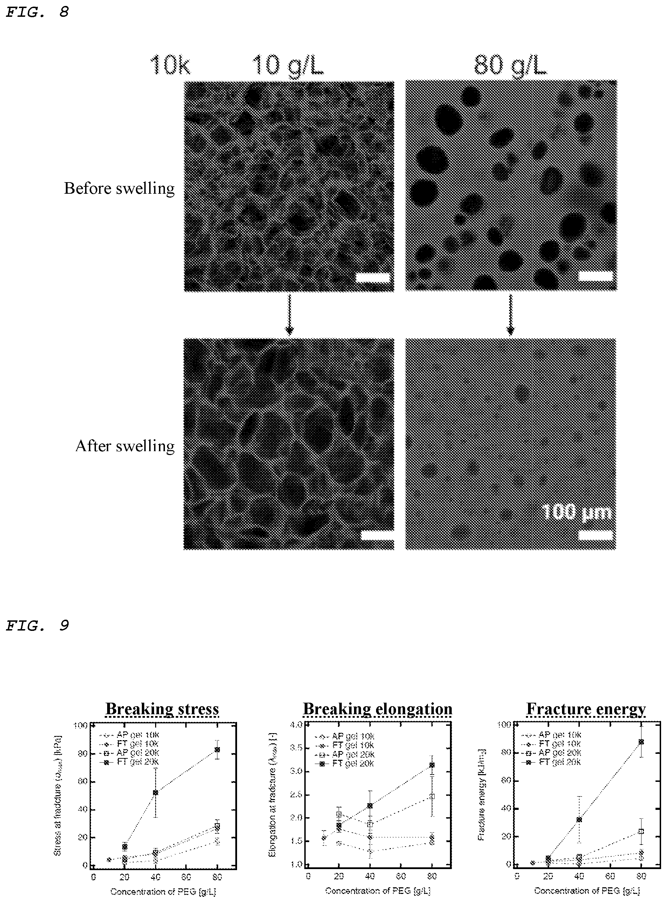

The obtained porous hydrogel was immersed in Dulbecco's phosphate buffered saline (D-PBS (-)) to evaluate the swelling behavior of the gel (Figs. 7 and 8).

In Fig. 7, the equilibrium swelling degree Q of the hydrogel was defined as (d_{eq}/dᵢₙᵢ)³ and plotted (Fig. 7). Here, "d_{eq}" is the diameter of the hydrogel at equilibrium, and "dᵢₙᵢ" is the diameter of the hydrogel immediately after production. As a result, it was found that the porous hydrogel ("FT Gel" in Fig. 7) after the freeze-thaw treatment swells less than the non-porous initial hydrogel ("AP Gel" in Fig. 7). Specifically, it was found that when the equilibrium swelling degree of the hydrogel having the porous structure prepared such that Cₜₒₜₐₗ (Concentration of PEG in the drawing) is 20 g/L is Q_{low} and the equilibrium swelling degree of the hydrogel having the porous structure prepared with arbitrary Cₜₒₜₐₗ is Q, a relational expression of Q<1.2*Q_{low} is satisfied.

Similarly, when the internal structure of the porous hydrogel before and after swelling was confirmed with the confocal laser microscope, it was confirmed that the porous structure was maintained even after swelling (Fig. 8).

### 6. Evaluation of Mechanical Strength of Hydrogel

Next, the obtained porous hydrogel was subjected to mechanical strength evaluation by a uniaxial tensile test and a uniaxial compression test.

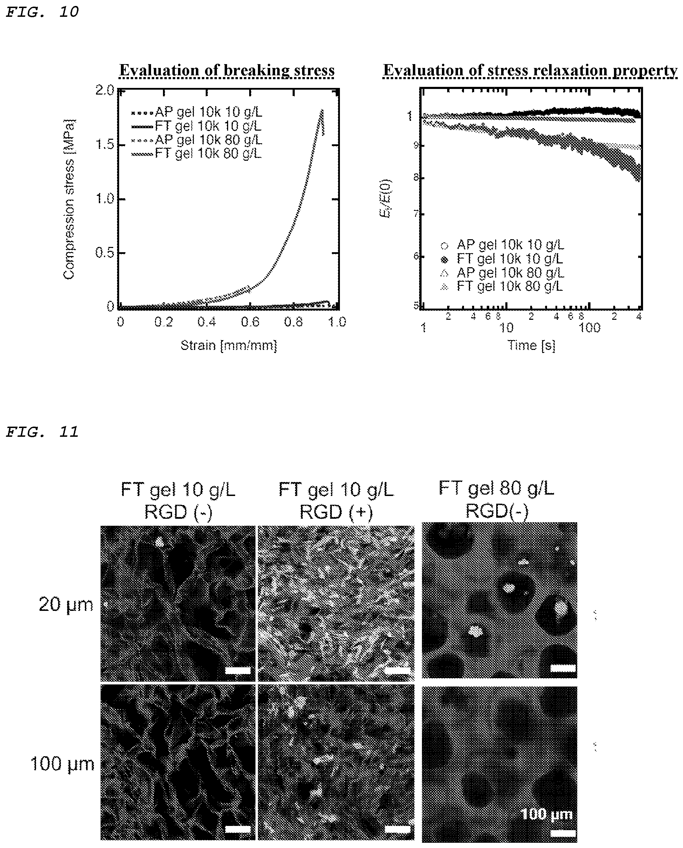

The uniaxial tensile test was performed with Autograph AG-X plus (Shimadzu Corporation), and the obtained measurement results of a breaking stress, breaking elongation, and fracture energy are illustrated in Fig. 9. The same test was performed five times for each hydrogel, and the average value and its standard deviation were plotted. In the figure, "AP Gel" represents a non-porous initial hydrogel, and "FT Gel" represents the porous hydrogel after the freeze-thaw treatment (the same applies to Fig. 10). As a result, it was confirmed that the porous hydrogel was improved in both strength and toughness.

Similarly, Fig. 10 illustrates the measurement results of the breaking stress and the stress relaxation property obtained by the uniaxial compression test. As a result, it was confirmed that the porous hydrogel withstands higher compression and exhibits remarkable stress relaxation property.

### 7. Application to cell culture

Cell culture was performed using the obtained porous hydrogel. First, in order to produce a gel for cell culture, the following two kinds of aqueous PEG solutions were produced. Solvents are all CPB 50 mM (pH 5) unless otherwise indicated.

Type 1) GRGDSPC peptide (GenScript) was mixed in a Tetra-PEG-MA solution so as to be 0.8 mM, and the mixture was incubated at 25°C for 5 minutes.

Type 2) Alexa Fluor^{™}594 C5 Maleimide (1 g/L DMSO solution) was mixed with a Tetra-PEG-SH solution so as to be 1% by volume, and the mixture was incubated at 25°C for 5 minutes.

These solutions were sterilized using a 0.22 µm filter. The filtered PEG solutions were mixed in the same volume, and the mixture was incubated in a rectangular silicon mold (15 mm × 35 mm, height: 3 mm) at 25°C for 24 hours. The formed gel was subjected to the freeze-thaw treatment. The freeze-thawed hydrogel was removed from the mold, immersed in D-PBS (-), and incubated at 25°C for 24 hours. The gel was further cut into a disk shape (diameter: 10 mm, thickness: 3 mm), placed in a 24 well plate, and then immersed in DMEM. Human dermal fibroblasts (passage number: 7) were cultured on a 10 cm dish at 37°C under 5% CO2. After a cell detachment treatment, the cells were seeded on the previously prepared disk-shaped gel, and cultured at 37°C under 5% CO2 for 7 days. According to the LIVE/DEADTM Viability/Cytotoxicity Kit product protocol, the cell culture gel was immersed in DMEM containing 1 µM calcein AM, and incubated at 37°C under 5% CO2 for 10 minutes. The hydrogel was washed with D-PBS (-) and then observed with the confocal laser microscope. As a result, good cell extension was confirmed in the porous gel (Fig. 11).

## Claims

1. A production method for a hydrogel having a porous structure, comprising the steps of:
a) preparing a first solution in which a compound A is dissolved in a first solvent;
b) preparing a second solution in which a compound B is dissolved in a second solvent;
c) obtaining an initial hydrogel having a non-porous structure by mixing the first solution and the second solution, the step comprising mixing the first solution and the second solution at a ratio such that a total polymer concentration (Cₜₒₜₐₗ) in a third solution obtained by mixing the first solution and the second solution is equal to or less than a higher one of two concentrations: a viscosity-increasing feature concentration (C_{A}) unique to the compound A or a viscosity-increasing feature concentration (C_{B}) unique to the compound B;
d) obtaining a frozen hydrogel by subjecting the initial hydrogel to a freezing treatment; and
e) obtaining a hydrogel having a porous structure by treating the frozen hydrogel at a temperature equal to or higher than a higher one of melting points of the first and second solvents,
wherein each of the initial hydrogel and the hydrogel having the porous structure forms a gel obtained by crosslinking the compound A and the compound B with each other,
each of the first and second solvents is an aqueous solvent; and
the compounds A and B are bi-, tri-, tetra-, or octa-branched polyethylene glycols (PEG) having one or more nucleophilic functional groups or electrophilic functional groups in total in a side chain or at a terminal.

2. The production method according to claim 1, wherein the compound A and/or the compound B has a weight average molecular weight of 4×10⁴ or less.

3. The production method according to claim 1, wherein the freezing treatment is performed at a temperature of - 10°C or lower for 1 hour or more.

4. The production method according to claim 1, wherein when a transmittance of the initial hydrogel is X, a transmittance of the frozen hydrogel is Y, and a transmittance of the hydrogel having the porous structure is Z, a relational expression of $X > Z > Y$ is satisfied.

5. The production method according to claim 1, wherein when an equilibrium swelling degree of the hydrogel having the porous structure prepared such that Cₜₒₜₐₗ is 20 g/L is Q_{low} and an equilibrium swelling degree of the hydrogel having the porous structure prepared with any Cₜₒₜₐₗ is Q, a relational expression of $Q < 1.2 * {Q}_{low}$ is satisfied.

6. The production method according to claim 1, wherein when a breaking stress at elongation (σₘₐₓ) of the initial hydrogel is Sᵢₙᵢₜᵢₐₗ and a breaking stress at elongation of the hydrogel having the porous structure is S, a relational expression of $S > {S}_{initial}$ is satisfied.

7. The production method according to claim 1, wherein when an elongation rate at break (λₘₐₓ) of the initial hydrogel is Tᵢₙᵢₜᵢₐₗ and an elongation rate at break of the hydrogel having the porous structure is T, a relational expression of $T > {T}_{initial}$ is satisfied.

8. The production method according to claim 1, wherein when a Young's modulus (Eₘₐₓ) of the initial hydrogel is Uᵢₙᵢₜᵢₐₗ and a Young's modulus of the hydrogel having the porous structure is U, a relational expression of $U > {U}_{initial}$ is satisfied.

9. The production method according to claim 1, wherein fracture energy of the hydrogel having the porous structure is higher than fracture energy of the initial hydrogel.

10. The production method according to claim 1, wherein the compound A is a bi-, tri-, tetra-, or octa-branched PEG having two or more nucleophilic functional groups in total in a side chain or at a terminal; and
the compound B is a bi-, tri-, tetra-, or octa-branched PEG having two or more electrophilic functional groups in total in a side chain or at a terminal.

11. The production method according to claim 1, wherein the nucleophilic functional group is selected from the group consisting of a thiol group and an amino group, and the electrophilic functional group is selected from the group consisting of a maleimidyl group, an N-hydroxy-succinimidyl (NHS) group, and a sulfosuccinimidyl group.
